(19) Europäisches Patentamt
European Patent Office
Office européen des brevets

(11) **EP 3 845 219 A1**

(12) **EUROPEAN PATENT APPLICATION**
published in accordance with Art. 153(4) EPC

(43) Date of publication:
**07.07.2021 Bulletin 2021/27**

(21) Application number: **19855419.8**

(22) Date of filing: **27.08.2019**

(51) Int Cl.:
*A61K 9/14* (2006.01)   *A61K 31/5383* (2006.01)
*A61K 45/00* (2006.01)   *A61K 47/04* (2006.01)
*A61K 47/26* (2006.01)   *A61K 47/32* (2006.01)
*A61K 47/36* (2006.01)   *A61K 47/38* (2006.01)
*A61P 31/04* (2006.01)

(86) International application number:
**PCT/JP2019/033583**

(87) International publication number:
**WO 2020/045456 (05.03.2020 Gazette 2020/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **28.08.2018 JP 2018159363**

(71) Applicant: **TOWA PHARMACEUTICAL CO., LTD.
Kadoma-shi,
Osaka 571-8580 (JP)**

(72) Inventors:
• **OKUDA, Yutaka
Kadoma-shi, Osaka 571-8580 (JP)**
• **SAEKI, Isamu
Kadoma-shi, Osaka 571-8580 (JP)**
• **HONJO, Tatsuya
Kadoma-shi, Osaka 571-8580 (JP)**
• **HIRAISHI, Keisuke
Kadoma-shi, Osaka 571-8580 (JP)**
• **YUMINOKI, Kayo
Kadoma-shi, Osaka 571-8580 (JP)**

(74) Representative: **Brann AB
P.O. Box 3690
Drottninggatan 27
103 59 Stockholm (SE)**

(54) **DRUG-CONTAINING PARTICLE**

(57) The present invention provides a method of efficiently manufacturing drug-containing particle which has a sharp peak in the particle size distribution, is highly spherical, and good in fluidity. The present invention relates to a manufacturing method of a drug-containing particle, the drug-containing particle comprising a raw material particle and a coating layer on the outside of the raw material particle, the raw material particle and/or the coating layer comprising at least one drug, wherein the manufacturing method comprises a granulation process that agitates a mixture comprising a core particle for powder coating formed by coating the raw material particle with a polymer and a drug and/or pharmaceutically acceptable additive while spraying a solvent which can dissolve the polymer on the mixture.

FIG.8

**Description**

TECHNICAL FIELD

The invention relates to a core particle formed by coating a raw material particle with a polymer, a drug-containing particle comprising the core particle, and a method of manufacturing the same.

BACKGROUND ART

For pharmaceutical products, spherical granules containing a drug are useful from the viewpoints of bitterness masking, elution control, and stabilization. For example, at the same particle diameter, spherical granules have a smaller surface area than non-spherical granules, and they can be coated more efficiently. Similarly, granules having a sharp peak in the particle size distribution can be coated more uniformly than granules having an even particle size distribution, and can be coated more efficiently. Such granules having the above-mentioned features can have a desired effect even with a reduced amount of coating agent; therefore, they are highly useful from the viewpoints of manufacturability and cost reduction.

In addition, because pharmaceutical formulations comprising spherical granules having a sharp peak in the particle size distribution have good flowability in the forms of granules, dry syrup, and the like, there are advantages that those products are excellent in productivity and easy to handle. Further, the spherical granules having a sharp peak in the particle size distribution have good flowability and also provide improved productivity in filling and tableting of capsules, tablets, and orally disintegrating tablets.

For obtaining spherical granules containing a drug, there is a known method in which a commercially available spherical additive is charged into a fluidized bed granulator or a tumbling granulator, which then sprays a solution or dispersion of a drug onto the spherical additive for coating. This method, however, has a disadvantage that it takes a long time for coating. In addition, the drug is kept being dissolved or dispersed in water or a solvent for a long time, which may cause a change in the crystal form of the drug or decomposition of the drug.

Further, there is another known method in which an additive and a drug in powder form are charged into a tumbling granulator, which then sprays a solution of a binder onto them for coating (see, for example, Patent Document 1); however, granules produced by this method does not have a sharp peak in the particle size distribution, and there is still room for improvement.

Further, there are other known methods for granulation in which smaller particles are adhered on the surface of core particles (see, for example, Patent Document 2).

PRIOR ART DOCUMENT

Patent Document

Patent Document 1: JP 2000-128774 A
Patent Document 2: JPH 06-218266 A

SUMMARY OF THE INVENTION

PROBLEM TO BE SOLVED BY THE INVENTION

An object of the present invention is to provide a method of efficiently manufacturing drug-containing particles which has a sharp peak in the particle size distribution, is highly spherical, and good in fluidity.

MEANS TO SOLVE THE PROBLEM

As a result of intensive studies, the inventors found that drug-containing particles having a desired particle diameter and particle size distribution can be manufactured in a short time by a simple granulation method where a drug and/or pharmaceutically acceptable additive in powder form is added to core particles formed by coating raw material particles with a polymer and the mixture is agitated while a solvent which can dissolve the polymer is sprayed on it.

To be specific, the present invention relates to:

[1] A core particle for powder coating by a wet method, wherein the core particle is formed by coating a raw material particle with a polymer;

[2] The core particle of claim 1, wherein the raw material particle comprises at least one selected from the group consisting of D-mannitol, lactose, crystalline cellulose, corn starch, silicon dioxide, low-substituted hydroxypropyl cellulose, carmellose, carmellose calcium, croscarmellose sodium, crospovidone, and a drug;

[3] The core particle of the above [1] or [2], wherein the polymer comprises at least one selected from the group consisting of hydroxypropyl cellulose, hypromellose, polyvinylpyrrolidone, polyvinyl alcohol, polyethylene glycol, pregelatinized starch, an ethyl acrylate-methyl methacrylate copolymer, an aminoalkyl methacrylate copolymer, a methacrylic acid copolymer, and ethyl cellulose;

[4] A manufacturing method of a drug-containing particle, the drug-containing particle comprising a raw material particle and a coating layer on the outside of the raw material particle, the raw material particle and/or the coating layer comprising at least one drug, wherein the manufacturing method comprises a granulation process that agitates a mixture comprising a core particle for powder coating formed by coating the raw material particle with a polymer and a drug and/or pharmaceutically acceptable additive while spraying a solvent which can dissolve the polymer on the mixture;

[5] The manufacturing method of the above [4], wherein the agitation granulation process is performed at a temperature of 35 to 100°C;

[6] The manufacturing method of the above [4] or [5], wherein the drug-containing particle comprises a discontinuity layer between the raw material particle and the coating layer;

[7] The manufacturing method of any one of the above [4] to [6], wherein the Span of the drug-containing particle is not more than 1.0;

[8] The manufacturing method of any one of the above [4] to [7], wherein the compressibility of the drug-containing particle is not more than 14%; and

[9] A drug-containing particle manufactured by the manufacturing method of any one of the above [4] to [8].

EFFECTS OF THE INVENTION

Use of the core particle for powder coating of the present invention improves efficiency in the process of powder coating onto core particles which conventionally has been a process taking a long time. In addition, the method is highly versatile and can be employed in manufacturing various solid formulations.

The manufacturing method of the present invention does not require preparation of a solution or suspension of a main drug and additive and allows use of a powder and a solvent as they are; therefore, the method is easy in operation, requires shorter manufacture time than a conventional core/shell granulation method, and hardly affects even main drug which is unstable in water or a solvent.

The manufacturing method of the present invention also can provide drug-containing particle with functionalities such as release control and bitterness masking, depending on the composition of the core particle. Further, it allows designing and manufacturing of drug-containing particle with a desired particle diameter and particle size distribution.

BRIEF DESCRIPTION OF THE DRAWINGS

FIG. 1 is an electron microscope photograph showing the appearance of raw material particle (CELPHERE CP-102Y).

Fig. 2 is an electron microscope photograph showing the appearance of core particle formed by coating the raw material particle (CELPHERE CP-102Y) with a water-soluble polymer (HPC-L).

Fig. 3A is an electron microscope photograph showing the appearance of the drug-containing particle of Example 1.

Fig. 3B is an electron microscope photograph showing the appearance of the drug-containing particle of Example 1.

Fig. 4A is an electron microscope photograph showing a section of the drug-containing particle of Example 1.

Fig. 4B is an electron microscope photograph showing a section of the drug-containing particle of Example 1.

Fig. 4C is an electron microscope photograph showing a section of the drug-containing particle of Example 1.

Fig. 5A is an electron microscope photograph showing the appearance of the drug-containing particle of Comparative Example 1.

Fig. 5B is an electron microscope photograph showing the appearance of the drug-containing particle of Comparative Example 1.

Fig. 6A is an electron microscope photograph showing the appearance of the drug-containing particle of Comparative Example 2.

Fig. 6B is an electron microscope photograph showing the appearance of the drug-containing particle of Comparative Example 2.

Fig. 7A is an electron microscope photograph showing the appearance of the drug-containing particle of Comparative Example 3.

Fig. 7B is an electron microscope photograph showing the appearance of the drug-containing particle of Comparative Example 3.

Fig. 8 is a graph showing the particle size distribution of the drug-containing particle of Example 1.

Fig. 9 is a graph showing the particle size distribution of the drug-containing particle of Comparative Example 1.

Fig. 10 is a graph showing the particle size distribution of the drug-containing particle of Comparative Example 2.

Fig. 11 is a graph showing the particle size distribution of the drug-containing particle of Comparative Example 3.

Fig. 12 is a graph showing the particle size distribution of the raw material particle (CELPHERE CP-102Y).

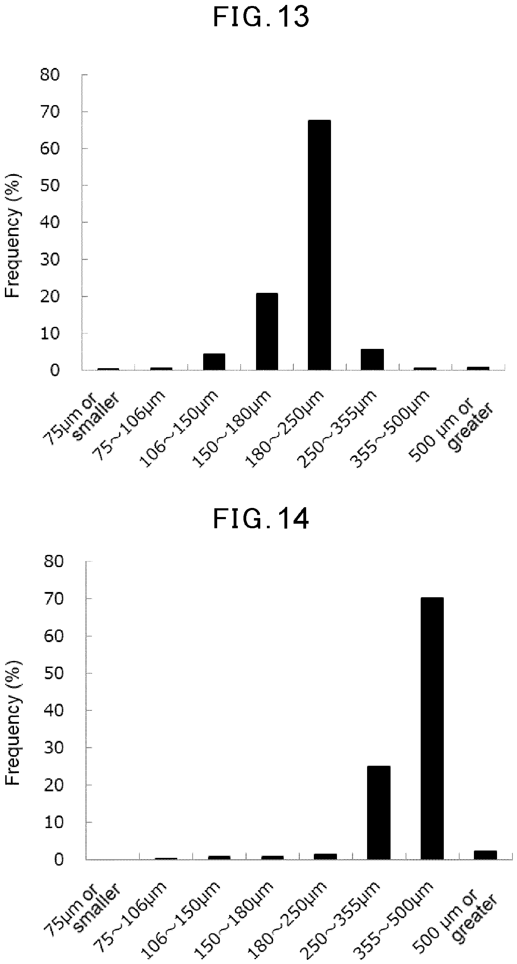

Fig. 13 is a graph showing the particle size distribution of the drug-containing particle of Example 2.

Fig. 14 is a graph showing the particle size distribution of the drug-containing particle of Example 3.

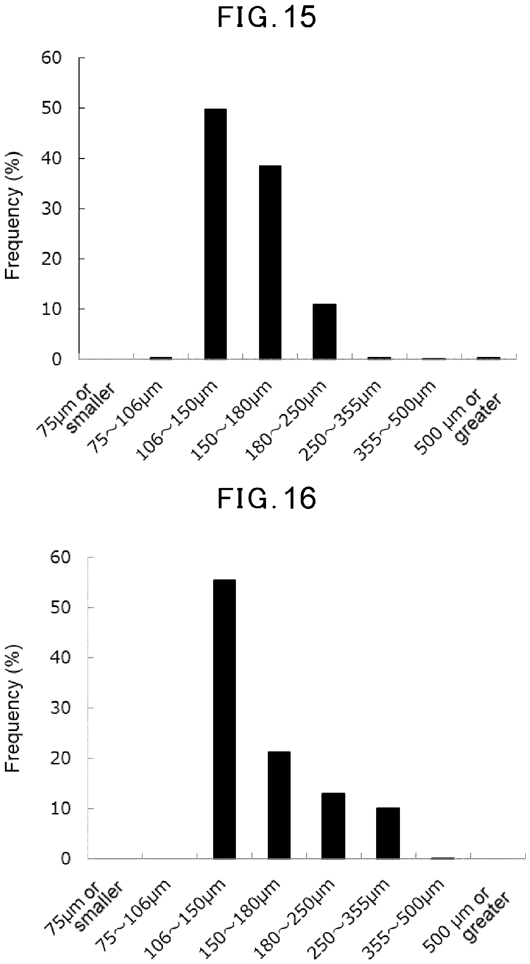

Fig. 15 is a graph showing the particle size distribution of the drug-containing particle of Example 4.

Fig. 16 is a graph showing the particle size distribution of the drug-containing particle of Example 5.

EMBODIMENT FOR CARRYING OUT THE INVENTION

In the following, steps of preparing drug-containing particle, including preparation of core particles for powder coating of one embodiment of the present invention are explained in detail. It should be noted, however, that the following description is exemplary for explaining the present invention and not intended to limit the technical scope of the present invention to this scope. Also, when "to" is used to indicate a numerical range, the range is inclusive at both ends.

The term "an average particle diameter" used herein refers to a particle diameter of 50% cumulative percentage (D50) on volume-based measurement of powder particle, which can be measured on the mass with a commercially available laser diffraction type particle size distribution measuring device and sonic wave vibration type sieving analyzer.

A compressibility, which herein indicates the flowability of particles, can be calculated from a specific volume (loose (mL/g), tap (mL/g)) that is measured with a 100mL stainless cup, by the following formula:

$$\text{(Compressibility (\%))} = \text{(loose (mL/g))} - \text{(tap (mL/g))}/\text{(loose (mL/g))} \times 100$$

A Span, which herein indicates the dispersion of particle size distribution, can be obtained from a particle diameter of 90% cumulative percentage (D90), a particle diameter of 10% cumulative percentage (D10), and a particle diameter of 50% cumulative percentage (D50) on mass-based measurement using a commercially available laser diffraction type particle size distribution measuring device or sonic wave vibration type sieving analyzer, by the following formula:

$$\text{(Span)} = (D90 - D10)/D50$$

<Core particle for powder coating and manufacturing method of the same>

The core particle for powder coating of the embodiment (which, hereinafter, may be just referred to as core particle) is characterized by being coated with a polymer having functionality. With such core particle having a polymer being localized on the surface of the raw material particle, spherical drug-containing particle desirably having a sharp peak in the particle size distribution can be manufactured in a short time by a simple granulation method where a drug and/or pharmaceutically acceptable additive in powder form is added and the mixture is agitated while a solvent which can dissolve the polymer is sprayed thereon.

When the polymer, which acts as a binder, exists irregularly, it not only binds the drug and/or the pharmaceutically acceptable additive with the raw material particle, but also binds the raw material particle together or the drug and/or the pharmaceutically acceptable additive together, which makes it hard to obtain granules having a sharp peak in the particle size distribution. It is considered that, in the core particle for powder coating of the embodiment, the polymer acting as a binder covers the raw material particle in advance so that it is used up to selectively bind the drug and/or the pharmaceutically acceptable additive with the raw material particle. Then a solvent which can dissolve the polymer is sprayed on the particles while it is agitated so that the polymer is gradually dissolved, and the drug and/or the pharmaceutically acceptable additive is adhered thereto, allowing efficient progress of spherical granulation.

(Raw material particle)

As raw material particle, a drug, a pharmaceutically acceptable additive or the mixture thereof, or granules obtained by a known granulation technique, and the like can be used. These kinds of raw material particle can be used singly or in combination of two or more of those. The component of the raw material particle and one component of the later-described polymer may be the same. It should be noted that the above-mentioned additive must not inhibit the effect of the drug (an active pharmaceutical ingredient) to be mixed, and preferably the mixing of the additive provides an additional function.

Examples of pharmaceutically acceptable additive include diluents, binders, disintegrators, lubricants, sweeteners, flavoring agents, coating agents, stabilizers, coloring agents, solubilizers, and plasticizers.

Examples of diluents include, but are not particularly limited to, D-mannitol, lactose, crystalline cellulose, pregelatinized starch, agar, gelatin, white sugar, corn starch, calcium hydrogen phosphate and silicon dioxide, and D-mannitol, lactose, crystalline cellulose, corn starch, and silicon dioxide are preferable.

Examples of binders include, but are not particularly limited to, hydroxypropyl cellulose, hypromellose (hydroxypropyl methyl cellulose), polyvinylpyrrolidone (povidone) and soybean lecithin, and hydroxypropyl cellulose and hypromellose are preferable.

Examples of disintegrators include, but are not particularly limited to, low-substituted hydroxypropyl cellulose, carmellose, carmellose calcium, carmellose sodium, croscarmellose sodium, crospovidone and carboxymethyl starch sodium. Low-substituted hydroxypropyl cellulose, carmellose, carmellose calcium, croscarmellose sodium and crospovidone are preferable, and low-substituted hydroxypropyl cellulose and crospovidone are more preferable.

Examples of lubricants include, but are not particularly limited to, talc, magnesium stearate, stearic acid, sucrose fatty acid ester and stearyl alcohol, and magnesium stearate is preferable.

Examples of sweeteners include, but are not particularly limited to, aspartame, saccharin and sucralose, and aspartame is preferable.

Examples of flavoring agents include, but are not particularly limited to, peppermint, 1-menthol, and peppermint is preferable.

Examples of coating agents include, but are not particularly limited to, an ethyl acrylate-methyl methacrylate copolymer, an aminoalkyl methacrylate copolymer, a methacrylic acid copolymer, ethyl cellulose and titanium oxide, and an ethyl acrylate-methyl methacrylate copolymer, ethyl cellulose and titanium oxide are preferable.

Examples of coloring agents include, but are not particularly limited to, yellow ferric oxide, iron sesquioxide and black iron oxide, and yellow ferric oxide and iron sesquioxide are preferable.

Examples of solubilizers include, but are not particularly limited to, polysorbate and macrogol, and polysorbate is preferable.

Examples of plasticizers include, but are not particularly limited to, light anhydrous silicic acid and hydrated silicon dioxide, and light anhydrous silicic acid is preferable.

The drug to be contained in the drug-containing particles is not particularly limited and can be used regardless of its property such as being basic, acid, amphoteric, and neutral and its solubility. Also, these drugs may be used singly or in combination two or more of those.

Among the above-mentioned raw material particles, raw material particle comprising at least one selected from the group consisting of D-mannitol, lactose, crystalline cellulose, corn starch, silicon dioxide, low-substituted hydroxypropyl cellulose, carmellose, carmellose calcium, croscarmellose sodium, crospovidone, and a drug is preferable; and raw material particle being at least one selected from the group consisting of D-mannitol, lactose, crystalline cellulose, corn starch, silicon dioxide, low-substituted hydroxypropyl cellulose, carmellose, carmellose calcium, croscarmellose sodium, crospovidone, and a drug is more preferable. Further, a combination of one selected from the group consisting of D-mannitol, lactose, crystalline cellulose, corn starch, silicon dioxide, low-substituted hydroxypropyl cellulose, carmellose, carmellose calcium, croscarmellose sodium, crospovidone, and a drug and a binder and/or a coating agent is also one preferable embodiment.

The average particle diameter of the raw material particles is arbitrary, and can be designed depending on the type of raw material particle, where, for example, it can be in the ranges of 5 to $1000\mu$m, 10 to 800pm, 20 to 600pm, 30 to 500pm, 40 to 400pm, 50 to 300pm.

The Span of the raw material particles is preferably not more than 1.0, more preferably not more than 0.9, further preferably not more than 0.8, and particularly preferably not more than 0.7.

(Polymer and core particle)

The core particle of the present embodiment is manufactured by coating the raw material particle with the polymer such as a water-soluble polymer and water-insoluble polymer. The polymer can be used singly or in combination of two or more of those.

Examples of water-soluble polymer include, but are not particularly limited to, cellulose derivatives and salts thereof such as methyl cellulose, hydroxypropyl cellulose (HPC), hypromellose (hydroxypropyl methyl cellulose), hydroxyethyl cellulose, hydroxymethyl cellulose, carboxymethylcellulose; water-soluble vinyl derivatives such as polyvinylpyrrolidone (povidone), polyvinyl alcohol, copolyvidone, polyethylene glycol (macrogol), a polyvinyl alcohol-acrylic acid-methyl methacrylate copolymer, a vinyl acetate-vinylpyrrolidone copolymer, polyvinyl alcohol-polyethylene glycol-graft copolymer; and pregelatinized starch, dextrin, dextran, pullulan, alginic acid, pectin, gelatin, hydrolysed gelatin, agar, polyvinyl alcohol, polyethylene glycol, pullulan, polysorbate 80, sodium lauryl sulfate, purified white sugar, and white sugar. Preferably, it is a water-soluble polymer containing at least one selected from the group consisting of hydroxypropyl cellulose, hypromellose, polyvinylpyrrolidone, polyvinyl alcohol, polyethylene glycol, and pregelatinized starch; and more preferably it is a water-soluble polymer being at least one selected from the group consisting of hydroxypropyl cellulose, hypromellose, polyvinylpyrrolidone, polyvinyl alcohol, polyethylene glycol, and pregelatinized starch; further preferably it is hydroxypropyl cellulose and/or polyethylene glycol. These water-soluble polymers can be used singly or in combination of two or more of those.

Examples of water-insoluble polymer include, but are not particularly limited to, an ethyl acrylate-methyl methacrylate copolymer, an aminoalkyl methacrylate copolymer, a methacrylic acid copolymer, ethyl cellulose, hydroxypropyl methyl cellulose acetate succinate, hypromellose phthalate ester, partially pregelatinized starch, hardened oil, synthetic wax, carnauba wax, stearyl alcohol, stearic acid, calcium stearate, glycerine fatty acid ester, sucrose fatty acid ester dextrin, soybean lecithin. Preferably, it is a water-insoluble polymer containing at least one selected from the group consisting of an ethyl acrylate-methyl methacrylate copolymer, an aminoalkyl methacrylate copolymer, a methacrylic acid copolymer, and ethyl cellulose; more preferably it is a water-insoluble polymer cellulose being at least one selected from the group consisting of an ethyl acrylate-methyl methacrylate copolymer, an aminoalkyl methacrylate copolymer, a methacrylic acid copolymer, and ethyl; further preferably it is ethyl cellulose and/or an aminoalkyl methacrylate copolymer; particularly preferably it is an aminoalkyl methacrylate copolymer. These water-insoluble polymers can be used singly or in combination of two or more of those.

An apparatus used for coating the raw material particle with a polymer is not particularly limited, and a fluidized bed granulator, a tumbling granulator, a tumbling fluidized bed granulator, a high-speed agitating granulator, a high-speed mixing and agitating granulator, a high-speed agitating mixer, a continuous spray granulator, a complex type fluidized bed granulator, and a dry particle composing machine is used.

The process of coating raw material particle with a polymer can be performed by, for example, charging raw material particle and a fluidization improving agent such as Aerosil into an above-mentioned complex type fluidized bed granulator, which then sprays a liquid in which a polymer is dissolved thereon.

The amount of polymer used for coating is, based on 100 parts by mass of the raw material particle, preferably 5 to 300 parts by mass, more preferably 10 to 200 parts by mass, further preferably 15 to 150 parts by mass, and particularly preferably 20 to 100 parts by mass.

<Drug-containing particle and a manufacturing method of the same>

The drug-containing particle of the present embodiment is characterized by having raw material particle and a coating layer on the outside of the raw material particle and comprising at least one drug in the raw material particle and/or the coating layer.

The drug-containing particles of the embodiment can be manufactured by a granulation process in which a solvent which can dissolve the polymer coating the raw material particle is added, for example, sprayed on a powdered mixture containing the above-mentioned core particles and a drug and/or pharmaceutically acceptable additive and the obtained granules is dried. That is, the core particle for powder coating of the present embodiment is suitably used in a wet core/ shell granulation method.

An apparatus for granulation of the drug-containing particles can be suitably selected, if it has an agitating function. There is no particular limitations of apparatus for granulation, but an agitating granulator, a high-speed agitating granulator, a mixing and agitating granulator, a high-speed mixing and agitating granulator, a high-speed agitating mixer, a tumbling granulator, a tumbling fluidized bed granulator are preferable. Among those, it is more preferable to use a high-speed agitating granulator, a mixing and agitating granulator, or a tumbling granulator.

Granulation of the drug-containing particle can be carried out under a heated condition. For example, it can be carried out at a temperature of 35 to 100°C, preferably 40 to 90°C.

A known drying method such as a shelf type dryer and drying by a fluidized bed can be suitably selected.

A "solvent" used herein refers to any solvent accepted in the fields such as pharmaceuticals, quasi-drugs, cosmetics, and foods and is not particularly limited if it can dissolve an above-mentioned polymer coating the raw material particle, but a solvent that is pharmaceutically acceptable is preferable.

Examples of solvents which can be used in the embodiment include water; alcoholic solvents such as methanol, ethanol, n-propanol and isopropanol; ketone solvents such as acetone and methyl ethyl ketone; ester solvents such as ethyl acetate; and mixed solvents of those.

When a water-soluble polymer is used as the polymer coating the raw material particle, water is suitably used; however, other solvents such as moisture ethanol can be used, which may be required for improving working property. In addition, when a water-insoluble polymer is used as the polymer coating the raw material particle, ethanol is used suitable; however, other solvents such as alcoholic solvents, ketone solvents, ester solvents can be used depending on the solubility.

The amount of the solvent to be used varies in accordance with types and volumes of the drug and the polymer, and is usually, based on 100 parts by weight of the total amount of the components of the drug-containing particles, 3 to 100 parts by weight, preferably 5 to 80 parts by weight, more preferably 5 to 60 parts by weight, further preferably 10 to 50 parts by weight.

For spraying the solvent, a spray gun which is normally used for granulation can be used. For increased yield of granules, the solvent is preferably sprayed as little as possible to the parts which are not the powder in a granulation vessel, for example, the inner wall of the granulation vessel and as wide as possible in the range of the powder in the granulation vessel.

Examples of pharmaceutically acceptable additives include, but are not limited to, pharmaceutically acceptable additives which can be used as the above-mentioned raw material particles. Also, even when the said additive is one of the above-mentioned polymers, if it is not dissolved in the solvent to be used, the additive does not function as the polymer of the embodiment and can still be mixed as an additive.

The Span of the drug-containing particles is preferably not more than 1.0, more preferably not more than 0.9, further preferably not more than 0.8, and particularly preferably not more than 0.7.

The average particle diameter of the core particles is preferably 5 times or greater than the average particle diameter of the drug and/or pharmaceutically acceptable additive, more preferably 10 times or greater, further preferably 15 times or greater, and particularly preferably 20 times or greater. When the average particle diameter of the drug and/or pharmaceutically acceptable additive is in the above ranges, the granulation of the drug-containing particles efficiently progresses.

Because the drug-containing particles of the present embodiment has a sharp peak in the particle size distribution and has a circular shape, they are good in flowability. The "good flowability" here means that the compressibility is not more than 14%. It is preferably not more than 12%, more preferably not more than 10%. When the drug-containing particles with good flowability are applied to various dosage forms of pharmaceutical products, the quality and manufacturability of the pharmaceutical products can be improved.

Further, in one embodiment, the drug-containing particle of the present embodiment has a discontinuity layer between the raw material particle and the coating layer. The "discontinuity layer" here refers to a laminar space existing between the raw material particle being at the center of the particle and the coating layer enclosing the raw material particle, which can be observed with, for example, an electron microscope or an optical microscope.

The drug-containing particle of the present embodiment is useful as a drug or a raw material for drugs and can be administered orally or parenterally. It should be noted that the dosage is suitably selected depending on the drug to be administered.

The drug-containing particle of the present embodiment may be various dosage forms in accordance with purpose of usage. For example, the drug-containing particle of the present embodiment can be used as they are in the forms such as granules, injections that are prepared at time of use, and an implanted dosage form. In addition, they can be mixed with an optional additive to form a tablet (including an orally disintegrating tablet) by tableting or to form a capsule by filling a capsule. They also can be used as a suspension (an aqueous suspension, an oil suspension), an emulsion, and the like.

EXAMPLE

The present invention will be explained in reference to examples; however, the present invention is not to be limited to the examples.

In the following, chemicals used in examples and comparative examples are shown.

Raw material particles: crystalline cellulose (CELPHERE CP-102Y manufactured by Asahi Kasei Corp.)

Water-soluble polymer: hydroxypropyl cellulose (HPC-L manufactured by Nippon Soda Co. Ltd.)

Compound A: levofloxacin 0.5 hydrate (Cas No.: 138199-71-0; pulverized product by jet mill; average particle diameter: $3.38\mu m$)

(Example 1)

525g of raw material particles was charged into a complex type fluidized bed granulator (Multiplex MP-01/SFP manufactured by Powrex Corporation) and a 3% water solution of a water-soluble polymer was sprayed at 2.9 g/mL on the particles under the conditions of 70°C of the supply air temperature and 1000 rpm of the number of rotations of the rotor. After spraying 262.5g of the water-soluble polymer and drying the particles until the exhaust gas temperature reached 50°C, it was screened with 60 mesh to obtain core particles (average particle diameter: $170\mu m$).

A compound A was added to the obtained core particles in accordance with the charged amount specified in Table 1, and a high-speed agitating granulator (EarthTechnica Co., Ltd., LFS-GS-2J) was used to conduct a granulation process for 15 minutes while spraying a 50% ethanol water solution under the manufacturing condition shown in Table 2 (note that charged amounts in Table 1 are based on the charged amount of the compound A). A fluidized bed granulator (Multiplex MP-01 manufactured by Powrex Corporation) was used to dry the granules until the exhaust gas temperature reached 40°C, and it was screened with 30 mesh to obtain drug-containing particles.

(Comparative Example 1)

Drug-containing particles were obtained in accordance with the charged amount specified in Table 1 by the same method as Example 1, except that the raw material particles were not coated by the water-soluble polymer and the water-soluble polymer and the compound A were added in powder form to the raw material particles.

(Comparative Example 2)

Drug-containing particles were obtained in accordance with the charged amount specified in Table 1 by the same method as Example 1, except that the raw material particles were not coated by the water-soluble polymer; the compound A were added in powder form to the raw material particles; and the water-soluble polymer was dissolved in a 50% ethanol water solution and sprayed on the particles.

(Comparative Example 3)

Drug-containing particles were obtained in accordance with the charged amount specified in Table 1 by the same method as Example 1, except that the water-soluble polymer was not used.

<Measurement of particle size distributions and indexes (Span) of the dispersion of particle size distribution>

Particle size distributions (D10, D50, and D90) of the raw material particles were measured based on the mass with a laser diffraction type particle size distribution measuring device (SALD-3000J manufactured by Shimadzu Corporation). Particle size distributions (D10, D50, D90) of the obtained drug-containing particles were measured based on the mass with a sonic wave vibration type sieving analyzer (Robot shifter PRS-95C manufactured by Seishin Enterprise Co., Ltd.). Spans were obtained by the following formula. Results are shown in Table 1 and Table 3. A smaller Span value indicates that the particle size distribution has a sharper peak.

$$(Span) = (D90\text{-}D10)/D50$$

<Appearance and section of particles>

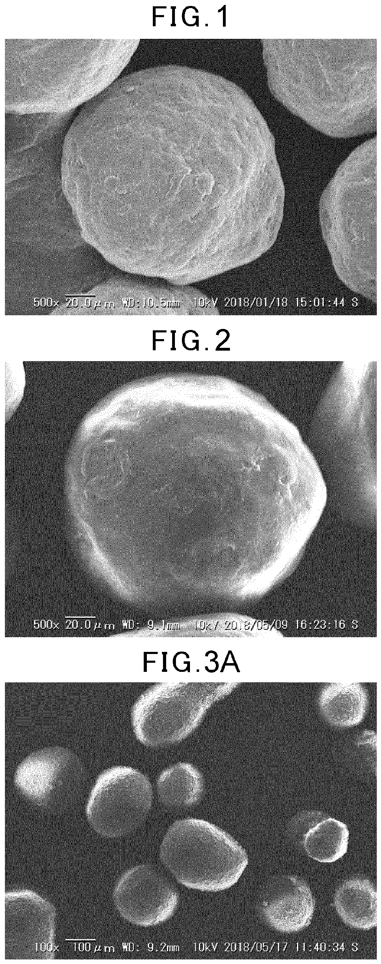

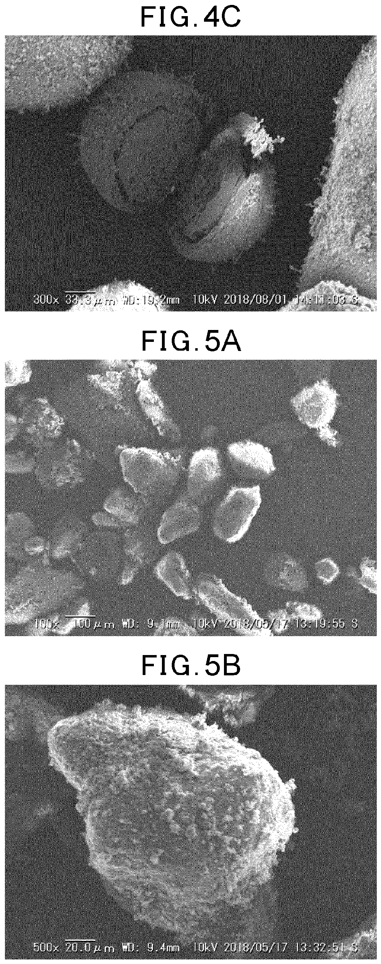

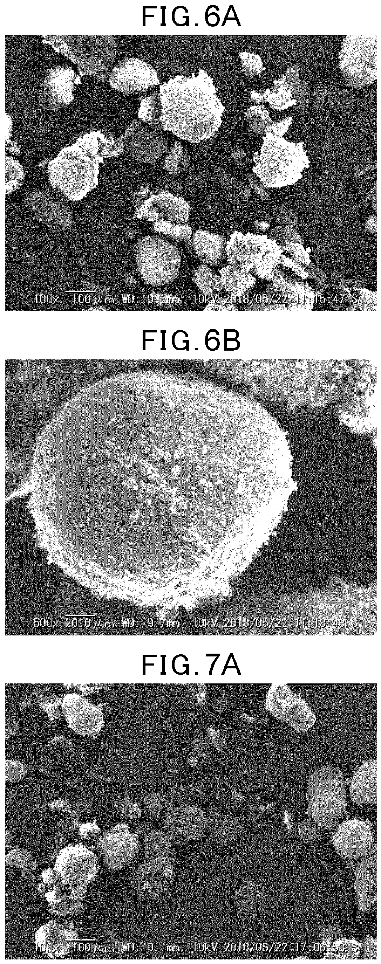

The appearance and section of the particles are observed with a scanning electron microscope (VE-7800 manufactured by Keyence Corporation) (Figures 1 to 7B).

<Measurement of specific volume and compressibility>

A specific volume is represented by the volume per unit weight of powder (mL/g). Drug-containing particles were charged to a 100mL stainless cup by naturally dropping them, the excess amount of the sample that was accumulated above the top of the stainless cup was scraped off using a plain metal plate, and subsequently a loose (mL/g) was calculated by measuring the mass of the stainless cup with the sample therein. Then the stainless cup was vibrated, and the drug-containing particles were again added thereto: this process was repeated until the process does not change the volume of the sample in the stainless cup. After the excess amount of the sample that was accumulated above the top of the stainless cup was scraped off using a plain metal plate, a tap (mL/g) was calculated by measuring the mass of the stainless cup with the sample therein. The compressibility (%) of the powder was calculated from the values of loose (mL/g) and tap (mL/g) by the following formula. Results were shown in Table 1. A lower compressibility indicates that the particles have better flowability.

$$(Compressibility\ (\%)) = ((loose\ (mL/g)) \text{ - } (tap\ (mL/g))/(loose\ (mL/g)) \times 100$$

Table 1

| | Example e | Comparative Examples | | |
|---|---|---|---|---|
| | 1 | 1 | 2 | 3 |
| **Charged amount (mg)** | | | | |
| Raw material particles | 62 | 62 | 62 | 73 |
| Water-soluble polymer | 31 (coating raw material particles) | 31 (added in powder form) | 31 (added in solution form) | - |
| Compound A | 125 | 125 | 125 | 145 |
| 50% ethanol water solution | 54 | 54 | 54 | 54 |
| **Particle size** | | | | |
| 32 mesh (500-710$\mu$m) (%) | 0.0 | 0.0 | 0.4 | 0.0 |
| 42 mesh (355-500$\mu$m) (%) | 7.3 | 6.8 | 14.1 | 8.5 |
| 60 mesh (250-355$\mu$m) (%) | 23.9 | 14.8 | 18.9 | 10.2 |
| 80 mesh (180-250$\mu$m) (%) | 59.0 | 20.8 | 20.3 | 7.0 |
| 100 mesh (150-180$\mu$m) (%) | 8.7 | 21.7 | 20.1 | 10.6 |
| 150 mesh (106-150$\mu$m) (%) | 1.1 | 29.8 | 20.5 | 47.2 |
| 200 mesh (75-106$\mu$m) (%) | 0.0 | 4.2 | 3.1 | 7.8 |
| N/A (75$\mu$m or smaller) (%) | 0.0 | 1.7 | 2.6 | 8.7 |
| D10 ($\mu$m) | 180 | 114 | 118 | 82 |
| D50 ($\mu$m) | 224 | 167 | 190 | 136 |
| D90 ($\mu$m) | 335 | 324 | 385 | 336 |
| Span | 0.69 | 1.25 | 1.41 | 1.87 |
| **Specific volume** | | | | |
| loose (mL/g) | 1.48 | 1.67 | 1.95 | 1.67 |
| tap (mL/g) | 1.40 | 1.31 | 1.56 | 1.42 |
| Compressibility (%) | 5.4 | 21.6 | 20.0 | 15.0 |

Table 2

| | Duration (min) | Method for adding solvent (solution) | Number of rotations of agitator (rpm) | Number of rotations of chopper (rpm) |
|---|---|---|---|---|
| **Processes** | | | | |
| Premixing | 3 | - | 400 | 400 |
| Granulation | 15 | Spraying (3.5g/min) | 400 to 140 | 3000 |

Table 3

| | Raw material particles |
|---|---|
| Particle size | |
| 32 mesh (500-710$\mu$m) (%) | 0.0 |
| 42 mesh (355-500$\mu$m) (%) | 0.0 |
| 60 mesh (250-355$\mu$m) (%) | 0.1 |
| 80 mesh (180-250$\mu$m) (%) | 0.2 |
| 100 mesh (150-180$\mu$m) (%) | 38.1 |
| 150 mesh (106-150$\mu$m) (%) | 61.6 |
| 200 mesh (75-106$\mu$m) (%) | 0.0 |
| N/A (75$\mu$m or smaller) (%) | 0.0 |
| D10 ($\mu$m) | 116 |
| D50 ($\mu$m) | 142 |
| D90 ($\mu$m) | 174 |
| Span | 0.41 |

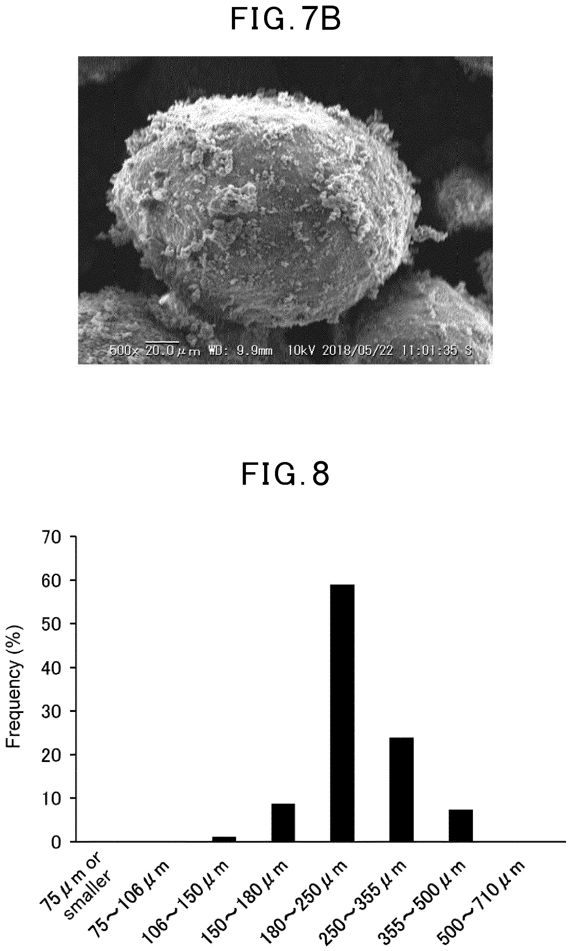

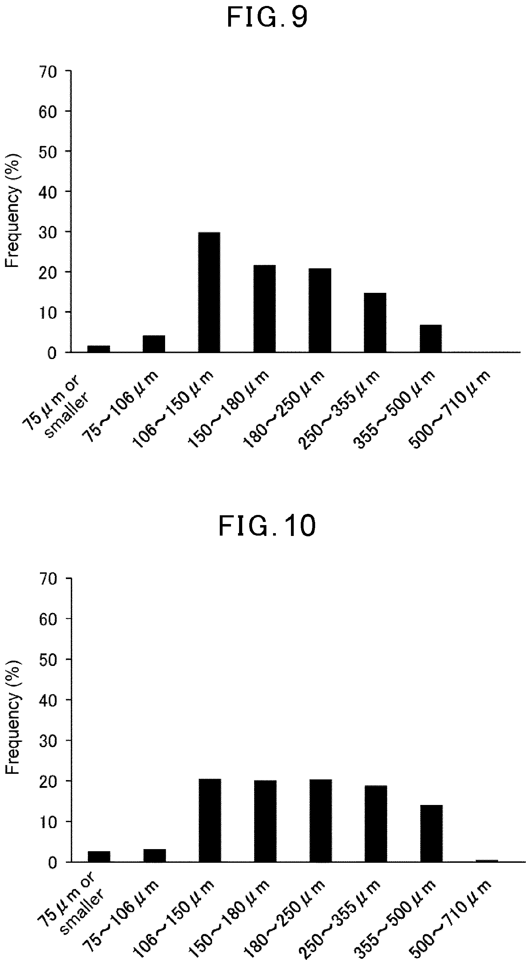

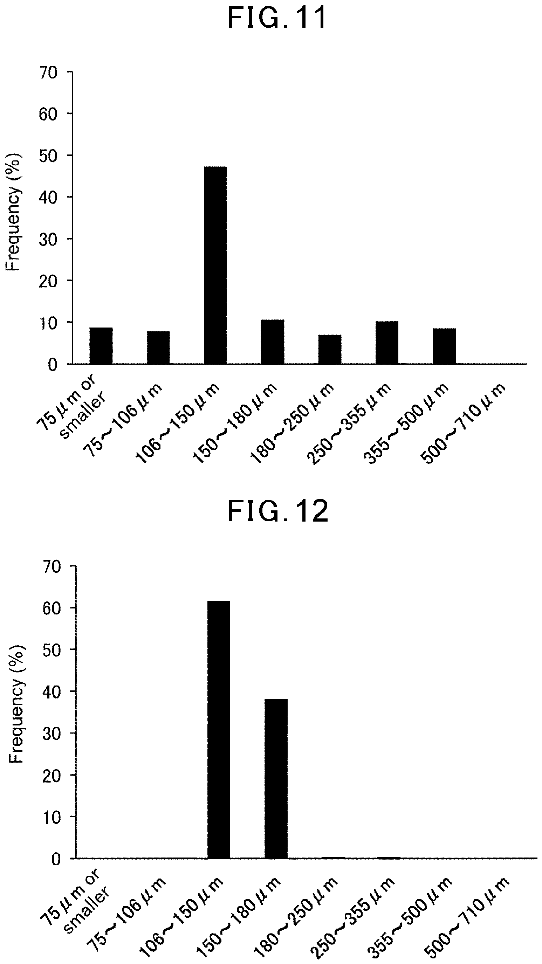

The results show the Span of the drug-containing particles of Example 1 is lower than 1.0 and has a sharp peak in the particle size distribution. The results also show that the particle size distribution of the raw material particles is near 100 to 150 mesh (106 to 180pm) (Figure 12), and the drug-containing particles of Example 1 has a peak near 80 mesh (180 to 250pm) (Figure 8), and the particles have grown, while the particle size distributions of the drug-containing particles of Comparative Examples 1 to 3 have a peak near 100 to 150 mesh (106 to 180pm) or are even (Figure 9, 10, and 11), implying granulation has not progressed or, even if it has, it has progressed nonuniformly.

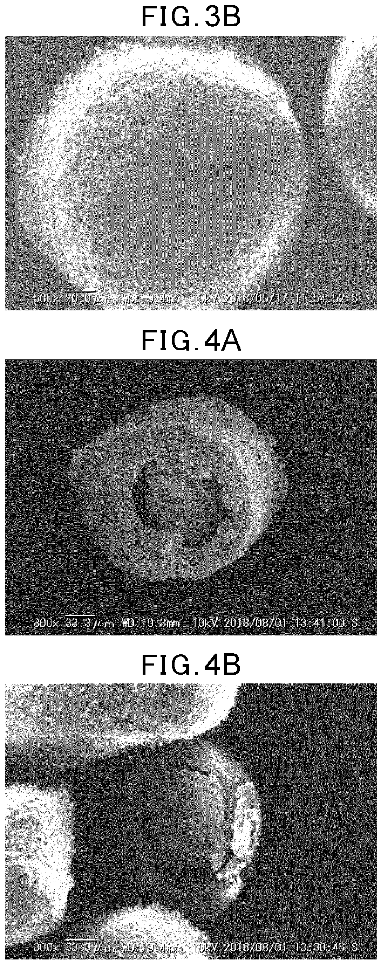

Further, observation by an electron microscope shows that the drug-containing particles of Example 1 are also highly spherical (Figure 3A to Figure 7B) and have a discontinuity layer. Further, it also shows that the drug-containing particles of Example 1 has a low compressibility and the flowability is improved.

(Example 2)

500g of raw material particles was charged into a complex type fluidized bed granulator (Multiplex MP-01 manufactured by Powrex Corporation/SFP) and a 3% water solution of a water-soluble polymer was sprayed at 2.9 g/mL on the particles under the conditions of 70°C of the supply air temperature and 1000 rpm of the number of rotations of the rotor. After spraying 150g of the water-soluble polymer and drying the particles until the exhaust gas temperature reached 50°C, it was screened with 60 mesh to obtain core particles (average particle diameter: 170$\mu$m).

A compound A was added to the obtained core particles in accordance with the charged amount specified in Table 4, and a high-speed agitating mixer (EL-1 manufactured by Nippon Eirich Co., Ltd.) was used to conduct a granulation process for 32 minutes while spraying a 50% ethanol water solution under the manufacturing condition shown in Table 5 (note that charged amounts on Table 4 are based on the charged amount of the compound A). A fluidized bed granulator (Multiplex MP-01 manufactured by Powrex Corporation) was used to dry the granules until the exhaust gas temperature reached 40°C to obtain drug-containing particles.

Table 4

| | Example |
|---|---|
| | 2 |
| Charged amount (mg) | |
| Raw material particles | 127.00 |
| Water-soluble polymer | 38.14 |
| Compound A | 152.50 |
| 50% ethanol water solution | 45.00 |
| Particle size | |
| 32 mesh (500-710$\mu$m) (%) | 0.7 |
| 42 mesh (355-500$\mu$m) (%) | 0.5 |
| 60 mesh (250-355$\mu$m) (%) | 5.6 |

(continued)

| Particle size | |
|---|---|
| 80 mesh (180-250$\mu$m) (%) | 67.4 |
| 100 mesh (150-180$\mu$m) (%) | 20.6 |
| 150 mesh (106-150$\mu$m) (%) | 4.4 |
| 200 mesh (75-106$\mu$m) (%) | 0.5 |
| N/A (75$\mu$m or smaller) (%) | 0.2 |
| D10 ($\mu$m) | 161 |
| D50 ($\mu$m) | 203 |
| D90 ($\mu$m) | 243 |
| Span | 0.41 |
| Specific volume | |
| loose (mL/g) | 1.40 |
| tap (mL/g) | 1.26 |
| Compressibility (%) | 10.0 |

Table 5

| | Duration (min) | Method for adding solvent (solution) | Number of rotations of rotor (rpm) | Number of rotations of vessel (rpm) |
|---|---|---|---|---|
| Processes | | | | |
| Premixing | 1 | - | 2400 | 85 |
| Granulation | 32 | Spraying (3.0g/min) | 7200 | 85 |

In the granulation of drug-containing particles, even when a high-speed agitating mixer was used instead of a high-speed agitating granulator, drug-containing particles having a sharp peak in the particle size distribution and being good in flowability were obtained in a short time.

(Example 3)

594g of crospovidone (Polyplasdone INF-10 manufactured by ISP Pharmaceuticals) and 6g of a water-soluble polymer were mixed with an agitation mixing granulator (Vertical Granulator FM-VG-05 manufactured by Powrex Corporation,) for one minute, and 613g of a 15% ethanol water solution was sprayed on the particles to obtain a kneaded object. A wet extrusion granulator Multigran (manufactured by Dalton Co., Ltd.: MG-55-2) equipped with a 0.3mm screen was used for extrusion granulation of the obtained kneaded object and Marumerizer spheronizer (manufactured by Dalton Co., Ltd.: QJ-230T-2) was used for sizing, and subsequently a fluidized bed granulator was used to dry it until the exhaust gas temperature reached 45°C. The obtained dried object was screened with sieves of 42 mesh (355$\mu$m) and 60 mesh (250$\mu$m), where those passing through the 42 mesh, but retained on the 60 mesh were disintegration core (average particle diameter: 298$\mu$m).

175g of the above-mentioned disintegration core was charged into a complex type fluidized bed granulator (Multiplex MP-01 manufactured by Powrex Corporation/SFP) and a 3% water solution of the water-soluble polymer was sprayed at 2.9 g/mL on the disintegration core under the conditions of 70°C of the supply air temperature and 1000 rpm of the number of rotations of the rotor. After spraying 52.5g of the water-soluble polymer and drying the particles until the exhaust gas temperature reached 50°C, those which passed the 42 mesh and were retained on the 60 mesh were core particles (average particle diameter: 295$\mu$m).

A compound A was added to the obtained core particles in accordance with the charged amount specified in Table 6, and a high-speed agitating mixer (EL-1 manufactured by Nippon Eirich Co., Ltd.) was used to conduct a granulation process for 60 minutes while spraying a 50% ethanol water solution under the manufacturing condition shown in Table 7 (note that charged amounts on Table 6 are based on the charged amount of the compound A). A fluidized bed granulator (Multiplex MP-01 manufactured by Powrex Corporation) was used to dry the granules until the exhaust gas temperature reached 40°C to obtain drug-containing particles.

Table 6

| | Example |
|---|---|
| | 3 |
| Charged amount (mg) | |
| Crospovidone | 100 |
| Water-soluble polymer | 30 |
| Compound A | 120 |
| 50% ethanol water solution | 90 |
| Particle size | |
| 32 mesh (500-710μm) (%) | 2.2 |
| 42 mesh (355-500μm) (%) | 70.0 |
| 60 mesh (250-355μm) (%) | 25.0 |
| 80 mesh (180-250μm) (%) | 1.3 |
| 100 mesh (150-180μm) (%) | 0.6 |
| 150 mesh (106-150μm) (%) | 0.6 |
| 200 mesh (75-106μm) (%) | 0.3 |
| N/A (75μm or smaller) (%) | 0.0 |
| D10 (μm) | 292 |
| D50 (μm) | 399 |
| D90 (μm) | 473 |
| Span | 0.46 |
| Specific volume | |
| loose (mL/g) | 1.98 |
| tap (mL/g) | 1.80 |
| Compressibility (%) | 9.1 |

Table 7

| | Duration (min) | Method for adding solvent (solution) | Number of rotations of rotor (rpm) | Number of rotations of vessel (rpm) |
|---|---|---|---|---|
| Processes | | | | |
| Premixing | 1 | - | 2400 | 85 |
| Granulation | 60 | Spraying (3.5g/min) | 4800 | 85 |

Even when an object formed by granulation of a disintegrator (crospovidone) by an usual method was used as raw material particles instead of a diluent (crystalline cellulose), drug-containing particles having a sharp peak in the particle size distribution and being good in flowability were obtained in a short time.

(Example 4)

600g of cilostazol and 150g of a water-soluble polymer were added to 2500g of purified water so that a suspension of cilostazol was prepared. A continuous spray granulator (CTS-SGR manufactured by Powrex Corporation) was used to spray the suspension of cilostazol for granulation of cilostazol, the obtained granules were sized with 83 mesh (180μm) and 140 mesh (106μm) and those which passed the 83 mesh and was retained on the 140 mesh were drug core (average particle diameter: 137μm).

280g of the above drug core was charged into a complex type fluidized bed granulator (Multiplex MP-01 manufactured by Powrex Corporation/SFP) and a 3% water solution of the water-soluble polymer was sprayed at 2.3 g/mL on the drug core under the conditions of 75°C of the supply air temperature and 1000 rpm of the number of rotations of the rotor. After spraying 84g of the water-soluble polymer and drying the particles until the exhaust gas temperature reached 50°C, it was screened with 83 mesh and 140 mesh
to obtain core particles (average particle diameter: 141μm).

A talc (victory light SK-C manufactured by Shokozan Mining Co., Ltd.; average particle diameter: 3.23μm)) was added to the obtained core particles in accordance with the charged amount specified in Table 8, and a high-speed agitating mixer (EL-1 manufactured by Nippon Eirich Co., Ltd.) was used to conduct a granulation process for 80 minutes while spraying a 50% ethanol water solution under the manufacturing condition shown in Table 9 (note that charged amounts on Table 8 are based on the charged amount of the compound A). A fluidized bed granulator (Multiplex MP-01 manufactured by Powrex Corporation) was used to dry the granules until the exhaust gas temperature reached 40°C to obtain drug-containing particles.

Table 8

|  | Example |
|---|---|
|  | 4 |
| **Charged amount (mg)** |  |
| Cilostazol | 84.6 |
| Water-soluble polymer | 25.4 |
| Talc | 101.6 |
| 50% ethanol water solution | 35.0 |
| **Particle size** |  |
| 32 mesh (500-710μm) (%) | 0.3 |
| 42 mesh (355-500μm) (%) | 0.1 |
| 60 mesh (250-355μm) (%) | 0.2 |
| 80 mesh (180-250μm) (%) | 10.9 |
| 100 mesh (150-180μm) (%) | 38.5 |
| 150 mesh (106-150μm) (%) | 49.8 |
| 200 mesh (75-106μm) (%) | 0.2 |
| N/A (75μm or smaller) (%) | 0.0 |
| D10 (μm) | 115 |
| D50 (μm) | 150 |
| D90 (μm) | 190 |
| Span | 0.50 |
| **Specific volume** |  |
| loose (mL/g) | 1.28 |
| tap (mL/g) | 1.11 |
| Compressibility (%) | 13.3 |

Table 9

|  | Duration (min) | Method for adding solvent (solution) | Number of rotations of rotor (rpm) | Number of rotations of vessel (rpm) |
|---|---|---|---|---|
| **Processes** |  |  |  |  |
| Premixing | 1 | - | 2400 | 85 |
| Granulation | 80 | Spraying (1.4g/min) | 4800 | 85 |

Even when an object formed by granulation of a drug (cilostazol) by an usual method was used as raw material particles instead of a diluent (crystalline cellulose), drug-containing particles having a sharp peak in the particle size distribution and being good in flowability were obtained in a short time.

(Example 5)

A dry particle composing machine (Nob-Mini manufactured by Hosokawa Micron Corporation) was used to mix raw material particles and polyethylene glycol (macrogol 6000 manufactured by Sanyo Chemical Industries, Ltd.) for one minute, and subsequently a dry composing process was conducted under the condition of 40°C of the jacket temperature, with the number of rotations of the rotor being adjusted so that the motor load power was around 100W. The obtained composite particles were sieved with 83 mesh (180μm) and 140 mesh (106μm), and those which passed through the 83 mesh and was retained on the 140 mesh were core particles (average particle diameter: 130μm).

A compound A was added to the obtained core particles in accordance with the charged amount specified in Table 10, and a high-speed agitating granulator (EarthTechnica Co., Ltd., LFS-GS-2J) was used to conduct a granulation process for 30 minutes while spraying a 50% ethanol water solution under the manufacturing condition shown in Table 11 (note that charged amounts on Table 10 are based on the charged amount of the compound A). A fluidized bed granulator (Multiplex MP-01 manufactured by Powrex Corporation) was used to dry the granules until the exhaust gas temperature reached 40°C, and it was sieved with 42 mesh (355μm) to obtain drug-containing particles.

Table 10

|  | Example |
|---|---|
|  | 5 |
| Charged amount (mg) | |
| Raw material particles | 155 |
| Polyethylene glycol | 15 |
| Compound A | 60 |
| 50% ethanol water solution | 30 |
| Particle size | |
| 32 mesh (500-710μm) (%) | 0.0 |
| 42 mesh (355-500μm) (%) | 0.1 |
| 60 mesh (250-355μm) (%) | 10.1 |
| 80 mesh (180-250μm) (%) | 13.0 |
| 100 mesh (150-180μm) (%) | 21.3 |
| 150 mesh (106-150μm) (%) | 55.5 |
| 200 mesh (75-106μm) (%) | 0.0 |
| N/A (75μm or smaller) (%) | 0.0 |
| D10 (μm) | 115 |
| D50 (μm) | 145 |
| D90 (μm) | 252 |
| Span | 0.95 |
| Specific volume | |
| loose (mL/g) | 1.32 |
| tap (mL/g) | 1.22 |
| Compressibility (%) | 7.6 |

Table 11

|  | Duration (min) | Method for adding solvent (solution) | Number of rotations of agitator (rpm) | Number of rotations of chopper (rpm) | Jacket temperature (°C) |
|---|---|---|---|---|---|
| Processes | | | | | |
| Premixing | 2 | | 142 | 3000 | 40 |
| Granulation | 30 | Spraying (2. 1g/min) | 142 to 400 | 3000 | 40 |

Even when polyethylene glycol was used as water-soluble polymer instead of hydroxypropyl cellulose, drug-containing particles having a sharp peak in the particle size distribution and being good in flowability were obtained in a short time.

INDUSTRIAL APPLICABILITY OF THE INVENTION

The present invention can manufacture drug-containing particles having a sharp peak in the particle size distribution being highly spherical, and good in flowability can be manufactured efficiently, which can be employed for manufacturing various kinds of solid formulations

**Claims**

1. A core particle for powder coating by a wet method, wherein the core particle is formed by coating a raw material particle with a polymer.

2. The core particle of claim 1, wherein the raw material particle comprises at least one selected from the group consisting of D-mannitol, lactose, crystalline cellulose, corn starch, silicon dioxide, low-substituted hydroxypropyl cellulose, carmellose, carmellose calcium, croscarmellose sodium, crospovidone, and a drug.

3. The core particle of claim 1 or 2, wherein the polymer comprises at least one selected from the group consisting of hydroxypropyl cellulose, hypromellose, polyvinylpyrrolidone, polyvinyl alcohol, polyethylene glycol, pregelatinized starch, an ethyl acrylate-methyl methacrylate copolymer, an aminoalkyl methacrylate copolymer, a methacrylic acid copolymer, and ethyl cellulose.

4. A manufacturing method of a drug-containing particle,
the drug-containing particle comprising a raw material particle and a coating layer on the outside of the raw material particle,
the raw material particle and/or the coating layer comprising at least one drug,
wherein the manufacturing method comprises a granulation process that agitates a mixture comprising a core particle for powder coating formed by coating the raw material particle with a polymer and a drug and/or pharmaceutically acceptable additive while spraying a solvent which can dissolve the polymer on the mixture.

5. The manufacturing method of claim 4, wherein the agitation granulation process is performed at a temperature of 35 to 100°C.

6. The manufacturing method of claim 4 or 5, wherein the drug-containing particle comprises a discontinuity layer between the raw material particle and the coating layer.

7. The manufacturing method of any one of claims 4 to 6, wherein the Span of the drug-containing particle is not more than 1.0.

8. The manufacturing method of any one of claims 4 to 7, wherein the compressibility of the drug-containing particle is not more than 14%.

9. A drug-containing particle manufactured by the manufacturing method of any one of claims 4 to 8.

# FIG.1

# FIG.2

# FIG.3A

## FIG.3B

## FIG.4A

## FIG.4B

FIG. 4C

FIG. 5A

FIG. 5B

## FIG.6A

## FIG.6B

## FIG.7A

# FIG.7B

# FIG.8

# FIG. 9

# FIG. 10

## FIG.11

## FIG.12

# FIG. 13

# FIG. 14

## FIG. 15

## FIG. 16

## INTERNATIONAL SEARCH REPORT

| International application No. |
| --- |
| PCT/JP2019/033583 |

### A. CLASSIFICATION OF SUBJECT MATTER

Int.Cl.  A61K9/14(2006.01)i,  A61K31/5383(2006.01)i,  A61K45/00(2006.01)i,
         A61K47/04(2006.01)i,  A61K47/26(2006.01)i,  A61K47/32(2006.01)i,
         A61K47/36(2006.01)i, A61K47/38(2006.01)i, A61P31/04(2006.01)i

According to International Patent Classification (IPC) or to both national classification and IPC

### B. FIELDS SEARCHED

Minimum documentation searched (classification system followed by classification symbols)

Int.Cl.  A61K9/14,   A61K31/5383,   A61K45/00,   A61K47/04,   A61K47/26,
         A61K47/32, A61K47/36, A61K47/38, A61P31/04

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
    JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2015-221781 A (TAISHO PHARMACEUTICAL CO., LTD.) 10 December 2015, claims, paragraphs [0013]-[0017], examples, comparative examples 1, 4 (Family: none) | 1–9 |
| X | JP 4-338324 A (SHIONOGI & CO., LTD.) 25 November 1992, claims, paragraphs [0009]-[0017], examples (Family: none) | 1–9 |
| X | JP 2004-339162 A (SHIN-ETSU CHEMICAL CO., LTD.) 02 December 2004, claims, paragraphs [0011]-[0018], examples & US 2004/0228916 A1, claims, paragraphs [0014]-[0023], examples & EP 1477161 A1 | 1–9 |

☒  Further documents are listed in the continuation of Box C.      ☐  See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 05 November 2019 (05.11.2019) | 12 November 2019 (12.11.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/033583 |

C (Continuation).    DOCUMENTS CONSIDERED TO BE RELEVANT

| Category* | Citation of document, with indication, where appropriate, of the relevant passages | Relevant to claim No. |
|---|---|---|
| X | JP 2007-197373 A (AKIYAMA JOZAI CO., LTD.) 09 August 2007, claims, paragraphs [0021]-[0022], examples (Family: none) | 1-3 |
| X | JP 2-174931 A (TAKEDA PHARMACEUTICAL COMPANY LIMITED) 06 July 1990, claims, examples & US 5855914 A, claims, examples & EP 361874 A2 | 1-3 |
| A | JP 2006-102558 A (POWREX CORP.) 20 April 2006, entire text (Family: none) | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000128774 A

- JP H06218266 A

**Non-patent literature cited in the description**

- *CHEMICAL ABSTRACTS,* 138199-71-0